# NEW EUROPEAN PATENT SPECIFICATION

(11) **EP 3 230 460 B2**
(45) Date of publication and mention of the opposition decision: **29.11.2023**
(45) Mention of the grant of the patent: 07.10.2020
(21) Application number: 15868327.6
(22) Date of filing: 14.12.2015
(51) Int. Cl.: C12N 15/85, C12N 9/22, C12N 15/113, C12N 15/52, A61K 48/00, C12Q 1/68

(54) **METHODS AND COMPOSITIONS FOR SELECTIVELY ELIMINATING CELLS OF INTEREST**
VERFAHREN UND ZUSAMMENSETZUNGEN ZUR SELEKTIVEN ELIMINIERUNG VON BESTIMMTEN ZELLEN
PROCÉDÉS ET COMPOSITIONS POUR L'ÉLIMINATION SÉLECTIVE DE CELLULES D'INTÉRÊT

(30) Priority: 12.12.2014 US 201462091431 P
(43) Date of publication of application: 18.10.2017
(73) Proprietor: Zhu, James, Cupertino, California 95014 (US)
(72) Inventor: ZHANG, Yi, Wilmette, Illinois 60091 (US); ZHU, James, Cupertino, CA 95014 (US)
(74) Representative: Straus, Alexander
(86) International application number: PCT/US2015/065421
(87) International publication number: WO 2016/094888

(56) References cited:
- WO-A1-2008/136656
- WO-A1-2010/117464
- WO-A1-2014/113493
- WO-A1-2016/011428
- WO-A2-2013/158309
- WO-A2-2014/059173
- WO-A2-2014/165707
- WO-A2-2014/172470
- WO-A2-2014/172470
- US-A1- 2011 130 444
- US-A1- 2013 326 645
- US-A1- 2013 326 645
- US-A1- 2015 071 946
- US-B1- 6 537 805
- L. CONG ET AL: "Multiplex Genome Engineering Using CRISPR/Cas Systems", SCIENCE, vol. 339, no. 6121, 3 January 2013 (2013-01-03), pages 819-823, XP055469277, US ISSN: 0036-8075, DOI: 10.1126/science.1231143
- WEIQIANG LI ET AL: "Safeguarding clinical translation of pluripotent stem cells with suicide genes", ORGANOGENESIS, vol. 9, no. 1, 1 January 2013 (2013-01-01) , pages 34-39, XP055461402, US ISSN: 1547-6278, DOI: 10.4161/org.24317
- MAGID H AMER: "Gene therapy for cancer: present status and future perspective", MOLECULAR AND CELLULAR THERAPIES, BIOMED CENTRAL LTD, LONDON, UK, vol. 2, no. 1, 10 September 2014 (2014-09-10), page 27, XP021198259, ISSN: 2052-8426, DOI: 10.1186/2052-8426-2-27
- ZHONG, BONAN ET AL.: 'Safeguarding nonhuman primate iPS cells with suicide genes' MOLECULAR THERAPY vol. 19, no. 9, 01 September 2011, pages 1667 - 1675, XP055454510 DOI: 10.1038/MT.2011.51
- WIRTH, THOMAS ET AL.: 'History of gene therapy' GENE vol. 525, no. 2, 23 April 2013, pages 162 - 169, XP028576972 DOI: 10.1016/J.GENE.2013.03.137
- LI et al.: "Safeguarding clinical translation of pluripotent stem cells with suicide genes", Organogenesis, vol. 9, no. 1, 2013, pages 34-39, DOI: 10.4161/org.24317
- ZHONG et al.: Safeguarding Nonhuman Primate iPS Cells With Suicide Genes Molecular Therapy, vol. 19, no. 9, 2011, pages 1667-1675,
- GAJ et al.: "ZFN, TALEN and CRISPR/Cas-based methods for genome engineering", Trends Biotechnol, vol. 31, no. 7, 2013, pages 397-405,
- CONG et al.: "multiplex Genome engineering using CRISPR/Cas Systems", Science, vol. 339, 2013, pages 819-823, DOI: 10.1126/science.1231143
- Rosner Karli: "DNase1: a new personalized therapy for cancer?", Expert Rev. Anticancer Ther., vol. 11, no. 7, 1 January 2011 (2011-01-01), pages 981-984,
- AMER: "Gene therapy for cancer: present status and future perspective", Molecular and Cellular Therapies, vol. 2, 2013, pages 1-19,
- Extracts from a sequence search for orthologs obtained from http//www.informatics.jax.org
- Gene Trees from comparative genomic searches from http://mart.ensembl.org/indey/html
- Cooper & Alder (2006) Cell 124. 815-822, (24.02.2006)
- Wikipedia extract (https.<7en.wikipedia org/wiki/Ex vivo)
- Luo et al. Nature Scientific Reports (2019) 9:1074

## Description

### FIELD OF THE INVENTION

The present disclosure generally relates to selective cell elimination.

### BACKGROUND OF THE INVENTION

Cancer is the leading cause of morbidity and mortality worldwide, with approximately 14 million new cases and 8.2 million cancer related deaths or 14.6% of all human deaths in 2012 (WHO website (November 2014) "Cancer"). The main feature of cancer is abnormal cell growth with the potential to invade or spread to other parts of the body. An ideal therapy for cancer is to completely eliminate all cancer cells, while leaving all healthy cells unharmed. WO 2014/172470 discloses a method of detecting a sequence variation in a cancer cell, such as a translocation sequence, at a defined locus of a cell and exerting an effect on the cell upon detection of the sequence variation by using a chimeric protein comprising a dead Cas protein and a caspase (suicide gene). However, cancer patients undergoing current systemic therapies suffer multiple side effects from nausea to infertility due to non-selectivity of therapeutic agents. Therefore, there is a continuing need to develop new therapeutics for selectively eliminating cancer cells but preserving non-cancer cells.

### BRIEF SUMMARY OF THE INVENTION

The present disclosure in general relates to compositions and methods which is suitable for selectively killing a target cell, without effecting a non-target cell.

In an aspect, the present application discloses a composition comprising a) a guide RNA that is capable of hybridizing with a target sequence in the genome of a target cell; b) a Cas protein; and c) a polynucleotide encoding a suicide gene. Said target sequence is specific to the target cell. In an embodiment, the target sequence comprising a breakpoint junction of a chromosome translocation in the target cell. The target cell is a cancer cell. In an embodiment, the Cas protein is a Cas9 protein. In an embodiment, the suicide gene is selected from the group consisting of viral thymidine kinase, cytosine deaminases, intracellular antibody against anti-oxidative enzymes (AOEs), bacterial introreductase, caspase and DNase. In an embodiment, the suicide gene is operably linked to a regulatory element. In an embodiment, the regulatory element is a minimal promoter. In an embodiment, the suicide gene is operably linked to a cancer-specific promoter.

In an aspect, the present application discloses a composition comprising one or more vectors comprising a) a first nucleotide sequence encoding a guide RNA that is capable of hybridizing with a target sequence in the genome of a target cell; b) a second nucleotide sequence encoding a Cas protein; and c) a third nucleotide sequence encoding a suicide gene; wherein the first, second and third nucleotide sequence are in the same or different vector(s). In an embodiment, said second nucleotide sequence encoding a Cas protein is operably linked to a cancer-specific promoter.

In an aspect, the present application discloses a composition comprising a) a first guide RNA that is capable of hybridizing with a first target sequence in the genome of a target cell; b) a second guide RNA that is capable of hybridizing with a second target sequence in the genome of the target cell; c) a Cas protein; and d) a polynucleotide encoding a suicide gene.

In another aspect, the present application discloses a composition comprising a) a first nucleotide sequence encoding a first guide RNA that is capable of hybridizing with a first target sequence in the genome of a target cell; b) a second nucleotide sequence encoding a second guide RNA that is capable of hybridizing with a second target sequence in the genome of the target cells; c) a third nucleotide sequence encoding a Cas protein; and d) a fourth nucleotide sequence encoding a suicide gene; wherein the first, second, third and fourth nucleotide sequence are in same or different vector.

In an aspect, the present application discloses a cell comprising one or more vectors comprising a) a first nucleotide sequence encoding a guide RNA that is capable of hybridizing with a target sequence in the genome of a target cell; b) a second nucleotide sequence encoding a Cas protein; and c) a third nucleotide sequence encoding a suicide gene; wherein the first, second and third nucleotide sequence are in same or different vector.

In an aspect, the present application discloses a composition comprising a) a first guide RNA that is capable of hybridizing with a first target sequence in the genome of a plurality of target cells; b) a second guide RNA that is capable of hybridizing with a second target sequence in the genome of the plurality of target cells; c) a Cas protein; and d) a polynucleotide encoding a suicide gene.

In another aspect, the present application discloses a composition comprising one or more vectors comprising a) a first nucleotide sequence encoding a first guide RNA that is capable of hybridizing with a first target sequence in the genome of a plurality of target cells; b) a second nucleotide sequence encoding a second guide RNA that is capable of hybridizing with a second target sequence in the genome of the plurality of target cells; c) a third nucleotide sequence encoding a Cas protein; and d) a fourth nucleotide sequence encoding a suicide gene; wherein the first, second, third and fourth nucleotide sequence are in same or different vector.

In yet another aspect, the present application discloses a method comprising introducing into a target cell a composition comprising a) a guide RNA that is capable of hybridizing with a target sequence in the genome of the target cell; b) a Cas protein; and c) a polynucleotide encoding a suicide gene. Said target sequence is absent in the genome of a control cell. The target cell is a cancer cell and the control cell is a healthy cell.

In another aspect, the present application discloses a method comprising contacting a target cell with one or more vectors comprising a) a first nucleotide sequence encoding a guide RNA that is capable of hybridizing with a target sequence in the genome of the target cell; b) a second nucleotide sequence encoding a Cas protein; and c) a third nucleotide sequence encoding a suicide gene; wherein the first, second and third nucleotide sequence are in same or different vector.

In an aspect, the present application discloses a method comprising contacting a plurality of target cells with a composition comprising a) a first guide RNA that is capable of hybridizing with a first target sequence in the genome of the plurality of target cells; b) a second guide RNA that is capable of hybridizing with a second target sequence in the genome of the plurality of target cells; c) a Cas protein; and d) a polynucleotide encoding a suicide gene.

In an aspect, the present application discloses a method comprising contacting a plurality of target cells with one or more vectors comprising a) a first nucleotide sequence encoding a first guide RNA that is capable of hybridizing with a first target sequence in the genome of the plurality of target cells; b) a second nucleotide sequence encoding a second guide RNA that is capable of hybridizing with a second target sequence in the genome of the plurality of target cells; c) a third nucleotide sequence encoding a Cas protein; and d) a fourth nucleotide sequence encoding a suicide gene; wherein the first, second, third and fourth nucleotide sequence are in same or different vector.

In an aspect, the present application discloses a method for treating cancer comprising contacting a cancer cell with one or more vectors comprising a) a first nucleotide sequence encoding a guide RNA that is capable of hybridizing with a target sequence in the genome of the cancer cell; b) a second nucleotide sequence encoding a Cas protein; and c) a third nucleotide sequence encoding a suicide gene; wherein the first, second and third nucleotide sequence are in same or different vector. In an embodiment, said target sequence is specific to the cancer cell.

In another aspect, the present application disclose a composition comprising a) a gene-targeting nuclease or a variant thereof; and b) a polynucleotide encoding a suicide gene; wherein the gene-targeting nuclease is capable of facilitating the integration of the suicide gene into a target sequence in the genome of a target cell. In an embodiment, the gene-targeting nuclease is a zinc-finger nuclease (ZFN), a transcription activator-like effector nuclease (TALEN), a Cas protein, or a meganuclease.

In an aspect, the present application discloses a guide RNA comprising a first nucleotide sequence that complement to a sequence in a first chromosome in a target cell; and a second nucleotide sequence that complement to a sequence in a second chromosome in the target cell.

In an aspect, the present application discloses a vector comprising (a) a polynucleotide encoding a suicide gene; (b) a first homology arm located at the upstream of the polynucleotide encoding the suicide gene, wherein the first homology arm comprises a sequence in a first chromosome in a target cell; and (c) a second homology arm located at the downstream of the polynucleotide encoding the suicide gene, wherein the second homology arm comprises a sequence in a second chromosome in the target cell.

In another aspect, the present disclosure relates a method for selectively eliminating a first cell in a cell population comprising at least the first cell and a second cell, the method comprising: a) identifying a locus in a genome of the first cell, said locus comprising a polynucleotide sequence, wherein the second cell does not comprise the polynucleotide sequence in its genome; and b) integrating a suicide gene at the locus to induce the cell death of the first cell. In certain embodiment, the first cell is a cancer cell.

In certain embodiments, the present disclosure provides a method of selectively eliminating or reducing cancer cells in a subject, comprising a) identifying a target locus in the genome of a cancer cell in a subject, said locus comprising a target polynucleotide sequence, wherein a healthy cell does not comprise the target polynucleotide sequence in its genome; and b) administering a composition to the subject, said composition comprising (i) a guide RNA that is capable of hybridizing with the target polynucleotide sequence, (ii) a Cas protein, and (iii) a suicide gene; c) integrating the suicide gene at the locus in the genome of the cancer cell; and d) inducing the cell death of the cancer cell.

These and other features, aspects, and advantages of the present invention will become better understood with regard to the following description, appended claims and accompanying drawings.

### BRIEF DESCRIPTION OF THE FIGURES

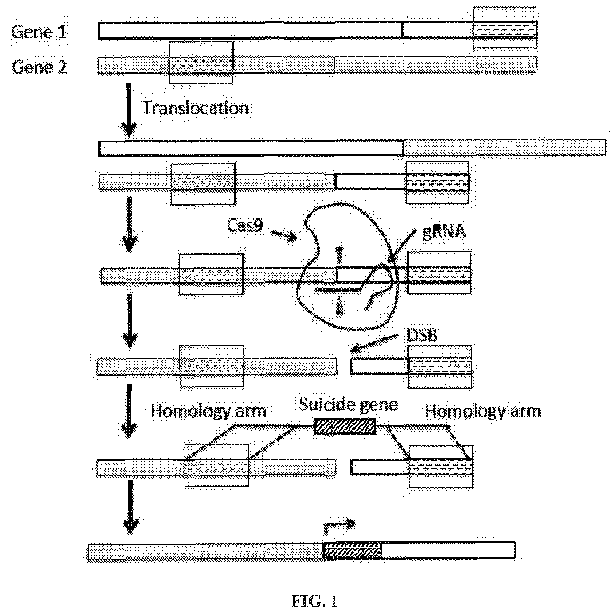

FIG. 1 is an illustration of inserting a suicide gene at a target sequence specific to a target cell that contains chromosome translocation. A DNA fragment of gene 1 is translocated to the locus of gene 2 and fuses to a DNA fragment of gene 2. A target sequence that specific to the cell that contains chromosome translocation is identified based on the sequence around the junction point.

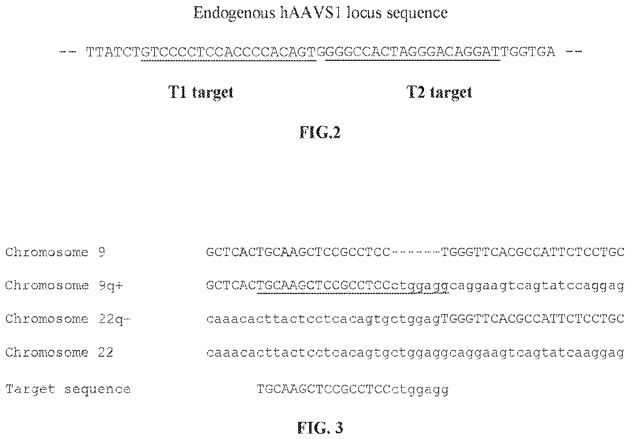

FIG. 2 illustrates the sequence of AAVS1 locus. Sequences used to design guide RNA (T1 and T2) are underlined.
FIG. 3 is an illustration of Junction sequences in t(9;22) in an ALL patient. Chromosome 9, 9q⁺, 22q⁻ and 22 represent sequences surrounding the breakpoint junction before or after the translocation. Chromosome 9 derived sequence is indicated by capitals; chromosome 22 derived sequence by lower case. Target sequence that contains Cas9 PAM sequence (underlined) is identified by a CRISPR gRNA design tool.

### DETAILED DESCRIPTION OF THE INVENTION

The term "comprises" and grammatical equivalents thereof are used herein to mean that other components, ingredients, steps, etc. are optionally present. For example, an article "comprising" (or "which comprises") components A, B, and C can consist of (i.e., contain only) components A, B, and C, or can contain not only components A, B, and C but also one or more other components.

Where reference is made herein to a method comprising two or more defined steps, the defined steps can be carried out in any order or simultaneously (except where the context excludes that possibility), and the method can include one or more other steps which are carried out before any of the defined steps, between two of the defined steps, or after all the defined steps (except where the context excludes that possibility).

Where a range of value is provided, it is understood that each intervening value, to the tenth of the unit of the lower limit unless the context clearly dictate otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

It will be appreciated that for simplicity and clarity of illustration, where appropriate, reference numerals have been repeated among the different figures to indicate corresponding or analogous elements. In addition, numerous specific details are set forth in order to provide a thorough understanding of the embodiments described herein. However, the embodiments described herein can be practiced without there specific details. In other instances, methods, procedures and components have not been described in detail so as not to obscure the related relevant function being described. Also, the description is not to be considered as limiting the scope of the implementations described herein. It will be understood that descriptions and characterizations of the embodiments set forth in this disclosure are not to be considered as mutually exclusive, unless otherwise noted.

The present disclosure relates to a composition that can be used to specifically kill a target cell. In an aspect, the present application discloses a composition comprising a) a guide RNA that is capable of hybridizing with a target sequence in the genome of a target cell; b) a Cas protein; and c) a polynucleotide encoding a suicide gene.

As used herein, a "target sequence" refers to a sequence to which a guide sequence is designed to have complementarity, where hybridization between a target sequence and a guide sequence promotes the formation of a CRISPR (Clustered Regularly Interspaced Short Palindromic Repeats) complex. The components of a CRISPR complex and the mechanism of using a CRISPR complex for gene editing has been described (e.g., M Jinek et al., Science, 2012, 337: 816-821; L Cong et al., Science, 2012, 339:819-823; PCT Publication WO2013176772, WO2013169802, WO2014018423 and US Patent No. 8,697,359) A target sequence can be any sequence in the genome of a target cell so long as the target sequence comprises a Protospacer Adjacent Motif (PAM) sequence, which is required by the formation of a CRISPR complex at the target sequence, at the 3' end of the target sequence. Exemplary target sequences include those that are unique in the genome of a target cell. For example, for the *S. pyogenes* Cas9, a unique target sequence in a genome may include a Cas9 target site of the form MMMMMMMMNNNNNNNNNNNNXGG where NNNNNNNNNNNNXGG (N is A, G, T, or C; and X can be any nucleotide) has a single occurrence in the genome. In this case, NNNNNNNNNNNN is complementary to a guid RNA and XGG is a PAM sequence. For the *S. thermophilus* CRISPR1Cas9, a unique target sequence in a genome may include a Cas9 target site of the form MMMMMMMMNNNNNNNNNNNNXXAGAAW where NNNNNNNNNNNNXXAGAAW (N is A, G, T, or C; X can be any nucleotide; and W is A or T) has a single occurrence in the genome. In each of these sequences "M" may be A, G, T, or C, and need not be considered in identifying a sequence as unique.

Said target sequence is specific to the genome of a target cell, i.e., the target sequence is absent in the genome of a control cell. As used herein, a "control cell" refers to a cell wherein the genome thereof lacks the target sequence in comparison to the genome of the target cell. The control cell is derived from the same species from which the target cell is derived. The control cell can be of the same or distinct type as the target cell. In some embodiments, the control cell is obtained from the same animal from which the target cell is obtained.
The target cell is a cancer cell and the control cell is a healthy cell.
In an embodiment, the cancer cell is obtained from a cancer patient. In an embodiment, both the cancer cell and the healthy cell are obtained from the same patient.

In some preferred embodiments, for a target sequence specific to a target cell, the target sequence embodies the following features: 1) the target sequence is specific to a target cell but absent in a non-target cell (for example a target sequence in a cancer cell is the region that results from chromosomal re-arrangement or translocation and thus is not present in non-cancer cells); 2) the target sequence includes a sequence complementary to a guide RNA sequence and a PAM sequence such that a CRISPR complex can be formed at the target sequence. Although not required, the target sequence preferably locates in an active or high-expression region of a chromosome in the target cell. It is conceivable that given the current genome sequence technology (Schuster SC, Next-generation sequencing transforms today's biology. Nat. Methods 2008, 5 (1): 16-8.), these sequences are either known in the public domain or readily sequenceable for any given target cell or target cell samples.

Numerous examples of DNA sequences specific to a target cell have been documented. In the context of a cancer cell, for example, uniquely aberrant DNA sequences caused by chromosome translocations have been described in certain cancers (e.g., acute myelogenous leukemia and chronic myelogenous leukemia). In such cancers, a gene fusion may be created when the translocation joins two otherwise-separated genes, resulting the over-expression of the gene fusion and abnormal proliferation of the cancer cells. Exemplified examples of cancer-associated translocation encompass those in Burkitt's lymphoma (c-myc on chromosome 8 is translocated to the immunoglobulin heavy locus on chromosome 14), Mantle cell lymphoma (cyclin D1 on chromosome 11 is translocated to immunoglobulin heavy locus), follicular lymphoma (bcl-2 is translocated to immunoglobulin heavy locus), follicular thyroid cancer (PAX-8 gene on chromosome 2 is translocated and fused with PPAR-gammal gene on chromosome 3), chronic myelogenous leukemia (CML) and acute lymphoblastic leukemia (Abl1 gene on chromosome 9 is translocated and fused with BCR (breakpoint cluster region) on chromosome 22). In each of these cases, target sequences specific to cancer cells can be identified on the basis of DNA sequences surrounding the breakpoint junction of the translocation (see, e.g., M J van der Feltz et al., Nucleic Acids Res., Jan 11, 1989; 17(1): 1-10; US Patent No. 4,874,853 to Rossi and 5,066,792 to Saunders et al.; and US PG Publication No. 20130209473 A1). In identifying a target sequence that can hybridize with a guide RNA, the target sequence can be chosen from a nucleotide sequence of about or more than about 5, 10, 11, 12, 13, 14, 15, 16, 17, 18, 19, 20, 21, 22, 23, 24, 25, 26, 27, 28, 29, 30, 35, 40, 45, 50, 75, or more nucleotides in length, encompassing the junction point of the translocation, and comprising the PAM sequence of a Cas protein at the 3' end of the sequence (e.g., XGG for *S. pyogenes* Cas9). Such target sequences can be identified using CRISPR gRNA design tools (e.g., Feng Zhang lab's target Finder, Michael Boutros lab's Targe Finder (E-CRISP), RGEN Tools (Cas-OFFinder), CasFinder, CRISPR Optimal Target Finder, DNA2.0). In one example, such target sequence is identified in the cancer cell from an ALL patient in which Abl1 gene on chromosome 9 is translocated and fused with BCR on chromosome 22. It is noted that the target specific sequence is not a sequence that exists in both target and non target cells (e.g., cancer gene that leads to the transition of normal cells into cancer cells). The target specific sequence is a sequence that is a result of cell development (e.g., growth of cancer) such that genes get mutated in cancer cells or re-arranged or translocated in cancer cell, both of which are not present in normal or non-cancer cells.

Methods of identifying the target sequence that is specific to a target cell is known in the art. Exemplified methods encompass PCR, southern blot, microarray and sequencing. In an embodiment, the genome of the target cell and the control cell can be sequenced and compared to identify a sequence that is specific to the target cell. For example, Garnett et al., has systematically identified mutated cancer genes in cancer cells (Nature, 2012, 433: 570-575). For another example, N Winhold et al, conducted a genome-wide analysis of noncoding regulatory mutations in cancer and systematically identified noncoding mutations in cancer (Nature Genetics, 2014, 46, 1160-1165). Other methods that can be used to identify a sequence specific to a target cell will occur to a person skill in the art.

In one example, a cancer patient is identified, and biopsy is performed to obtain the cancer cell from the patient. The genome of the cancer cell is then sequenced and compared with the genome of a healthy cell from the same patient. A sequence that exists only in the genome of the cancer cell and contains a PAM sequence at 3' end is then identified and is used as the target sequence when the features meet with the parameters set forth herein.

As used herein, a "guide RNA" refers to an RNA that directs sequence-specific binding of a CRISPR complex to the target sequence. Typically, a guide RNA comprises (i) a guide sequence that has sufficient complementarity with a target polynucleotide sequence to hybridize with the target sequence and (ii) a trans-activating cr (tracr) mate sequence. A guide RNA may further comprises a tracr RNA fused at the 3' end, resulting a single chimeric guide RNA. A CRISPR complex is formed in junction with a Cas, a guide RNA, a target sequence with PAM at the 3' end, and a tracr RNA (which can be a fused with the guide RNA or separated from the guide RNA). In some embodiments, the degree of complementarity between a guide sequence and its corresponding target sequence, when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences, non-limiting example of which include the Smith-Waterman algorithm, the Needleman-Wunsch algorithm, algorithms based on the Burrows-Wheeler Transform (e.g. the Burrows Wheeler Aligner), ClustalW, Clustal X, BLAT, Novoalign (Novocraft Technologies, ELAND (Illumina, San Diego, Calif), SOAP (available at soap.genomics.org.cn), and Maq (available at maq.sourceforge.net). In some embodiments, a guide sequence is about or more than about 5, 10, 11, 12, 13, 14, 15, 16, 17, 18, 19, 20, 21, 22, 23, 24, 25, 26, 27, 28, 29, 30, 35, 40, 45, 50, 75, or more nucleotides in length. In some embodiments, a guide sequence is less than about 75, 50, 45, 40, 35, 30, 25, 20, 15, 12, or fewer nucleotides in length. The ability of a guide sequence to direct sequence-specific binding of a CRISPR complex to a target sequence may be assessed by any suitable assay. For example, the components of a CRISPR system sufficient to form a CRISPR complex, including the guide sequence to be tested, may be provided to a host cell having the corresponding target sequence, such as by transfection with vectors encoding the components of the CRISPR sequence, followed by an assessment of preferential cleavage within the target sequence, such as by Surveyor assay as described herein. Similarly, cleavage of a target polynucleotide sequence may be evaluated in a test tube by providing the target sequence, components of a CRISPR complex, including the guide sequence to be tested and a control guide sequence different from the test guide sequence, and comparing binding or rate of cleavage at the target sequence between the test and control guide sequence reactions. Other assays are possible, and will occur to those skilled in the art.

In some embodiments, a guide sequence is selected to reduce the degree of secondary structure within the guide sequence. Secondary structure may be determined by any suitable polynucleotide folding algorithm. Some programs are based on calculating the minimal Gibbs free energy. An example of one such algorithm is mFold, as described by Zuker and Stiegler (Nucleic Acids Res. 9 (1981), 133-148). Another example folding algorithm is the online webserver RNAfold, developed at Institute for Theoretical Chemistry at the University of Vienna, using the centroid structure prediction algorithm (see e.g. A. R. Gruber et al., 2008, Cell 106(1): 23-24; and PA Carr and GM Church, 2009, Nature Biotechnology 27(12): 1151-62).

As used herein, a tracr mate sequence includes any sequence that has sufficient complementarity with a tracr sequence to promote one or more of: (1) excision of a guide sequence flanked by tracr mate sequences in a cell containing the corresponding tracr sequence; and (2) formation of a CRISPR complex at a target sequence, wherein the CRISPR complex comprises the tracr mate sequence hybridized to the tracr sequence. In general, degree of complementarity is with reference to the optimal alignment of the tracr mate sequence and tracr sequence, along the length of the shorter of the two sequences. Optimal alignment may be determined by any suitable alignment algorithm, and may further account for secondary structures, such as self-complementarity within either the tracr sequence or tracr mate sequence. In some embodiments, the degree of complementarity between the tracr sequence and tracr mate sequence along the length of the shorter of the two when optimally aligned is about or more than about 25%, 30%, 40%, 50%, 60%, 70%, 80%, 90%, 95%, 97.5%, 99%, or higher.

In some embodiments, the guide RNA comprises a guide sequence fused to a tracr sequence, i.e., the tracr sequence and tracr mate sequence are contained within a single transcript, such that hybridization between the two produces a transcript having a secondary structure, such as a hairpin. In some embodiments, the tracr sequence is about or more than about 5, 6, 7, 8, 9, 10, 11, 12, 13, 14, 15, 16, 17, 18, 19, 20, 25, 30, 40, 50, or more nucleotides in length. Preferred loop-forming sequences for use in hairpin structures are four nucleotides in length, and most preferably have the sequence GAAA. However, longer or shorter loop sequences may be used, as may alternative sequences. The sequences preferably include a nucleotide triplet (for example, AAA), and an additional nucleotide (for example C or G). Examples of loop forming sequences include CAAA and AAAG. In an embodiment of the present application, the guide RNA has at least two or more hairpins. In preferred embodiments, the guide RNA has two, three, four or five hairpins. In a further embodiment of the disclosure, the guide RNA has at most five hairpins. In some embodiments, the guide RNA further includes a transcription termination sequence, preferably a polyT sequence, for example six T nucleotides. In some embodiments, the tracr sequence is a separate transcript from a transcript comprising the tracr mate sequence. In certain embodiments, tracr sequence is in a separate vector from the guide RNA (see, e.g., US PG Pub No. 20140068797).

As used herein, a "Cas protein" refers a polypeptide that binds to the guide RNA and exhibit nuclease activity. Non-limiting examples of Cas proteins include Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2. Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, homologs thereof, or modified versions thereof. These enzymes are known; for example, the amino acid sequence of *S. pyogenes* Cas9 protein may be found in the SwissProt database under accession number Q99ZW2. In some embodiments, the unmodified Cas protein has DNA cleavage activity. In some embodiments, the Cas protein directs cleavage of one or both strands at the location of a target sequence, such as within the target sequence and/or within the complement of the target sequence. In some embodiments, the Cas protein directs cleavage of one or both strands within about 1, 2, 3, 4, 5, 6, 7, 8, 9, 10, 15, 20, 25, 50, 100, 200, 500, or more base pairs from the first or last nucleotide of a target sequence. In some embodiments, the Cas protein is mutated such that the mutated Cas protein lacks the ability to cleave one or both strands of a target polynucleotide containing a target sequence. For example, an aspartate-to-alanine substitution (D10A) in the RuvC I catalytic domain of Cas9 from *S. pyogenes* converts Cas9 from a nuclease that cleaves both strands to a nickase (cleaves a single strand). Other examples of mutations that render Cas9 a nickase include, without limitation, H840A, N854A, and N863A.

As used herein, the term Protospacer adjacent motif (PAM) refers to a DNA sequence immediately following the DNA sequence targeted by Cas protein. In some embodiments, PAM sequence is located at the 3' end of the target sequence and is required for the Cas protein to successfully bind to the target sequence. The PAM sequence varies by the species of the bacteria from which the Cas protein is derived. For example, the PAM sequence for Cas9 from *Steptococcuspyogenes* is NGG (N could be any of A, T, C or G). For another example, the PAM sequence for *Neisseria meningitides* is NNNNGATT. The PAM sequence for *Streptococcus thermophilic* is NNAGGAA. The PAM sequence for *Treponema denticola* is NAAAAC.

As used herein, the term "suicide gene" refers to a gene that causes a cell to kill itself. In some embodiments, the suicide gene causes a cell to kill itself through apoptosis. Non-limiting examples of suicide genes include viral thymidine kinase, cytosine deaminases, intracellular antibody against antioxidative enzymes (AOEs), bacterial nitroreductase, caspase and DNase (see Malecki M., Frontiers in Suicide Gene therapy of Cancer, J Genet Syndr Gene Ther. 2012(3), and references cited therein).

In one embodiment, the suicide gene is viral thymidine kinase (TK). Thymidine kinase is an ATP-thymidine 5'-phosphotransferase that converts deoxythymidine intodeoxythymidien 5'-monophosphate, which is further phosphorylated to deoxythymidine diphosphate and thereafter to deoxythymidine triphosphate by viral thymidine kinase and nucleoside diphosphate kinase respectively. Deoxythymidine triphosphate is incorporated into the synthesized DNA molecule by DNA polymerase. Some dNTP analogs, such as Ganciclovir (GCV), a synthetic analogue of 2'-deoxy-guanosine, have the ability to terminate the DNA synthesis upon their incorporation into synthesized DNA. Termination of synthesis triggers the apoptotic signaling cascades. While GCV is not recognized by human thymidine kinase, it is recognized as a substrate for some viral thymidine kinase, such as Herpes Simplex Virus-1 thymidine kinase (HSV-TK). As a result, a human cell expressing HSV-TK converts GCV into GCV phosphate, which is further phosphorylated and incorporated into the synthesized DNA, leading to the termination of synthesis and apoptosis.

In an embodiment, the suicide gene is cytosine deaminase. Cytosine deaminase hydrolyze cytosine to uracile with release of ammonia. In physiological conditions, the modified site is recognized by endonucleases, then the phophodiester bond in the DNA is broken, initiating repair by incorporation of a new cytosine. However, cytosine deaminase can also converts 5-fluorocytosine into 5-fluorouracil (5-FU). Therefore, upon provision of non-toxic prodrug 5-FC, cytosine deaminase converts it into highly toxic 5-FU (a suicide inhibitor of thymidylate synthetase), leading to the inhibition of cell growth and apoptosis.

In another aspect, the present application discloses a composition comprising one or more vectors comprising a) a first nucleotide sequence encoding a guide RNA that is capable of hybridizing with a target sequence in the genome of a target cell; b) a second nucleotide sequence encoding a Cas protein; and c) a third nucleotide sequence encoding a suicide gene; wherein the first, second and third nucleotide sequence are in same or different vector. In an embodiment, said second nucleotide sequence encoding the Cas protein is operably linked to a cancer-specific promoter.

As used herein, the term "vector" refers to a polynucleotide molecule that comprises a gene or a nucleic acid sequence of particular interest. Typically, the construct also includes appropriate regulatory sequences. For example, the polynucleotide molecule can include regulatory sequences located in the 5'-flanking region of the nucleotide sequence encoding the guide RNA and/or the nucleotide sequence encoding the Cas protein and/or the nucleotide sequence encoding a suicide gene, operably linked to the coding sequences in a manner capable of replicating and expressing the desired gene in a host cell.

In some embodiments, the vector comprising the suicide gene further comprises nucleotide sequences suitable for integration of the suicide gene to the target sequence. For example, the suicide gene is franked by two regions suitable for recombination around the target sequence. Typically, the first region has a high degree of homology to the sequence immediately upstream of the double strand break (DSB) created by the Cas protein, while the second region to the sequence downstream of the DSB. Upon the creation of a DSB by the Cas at the target sequence, the homolog-directed repair (HDR) machinery endogenous to the target cell is recruited to the loci of DSB and uses the vector as a suitable template to faithfully introduce the suicide gene at the DSB. In an embodiment, where the target sequence is created by a chromosome translocation in the target cell, the sequence upstream of the DSB is from one chromosomal, and the sequence downstream of the DSB is from another chromosome. As such, the vector will not be used as a template for the HDR machinery in a non-targeted cell, which does not contain a target sequence specific to the target cell.

The vector comprising the nucleotide sequence encoding the guide RNA and/or the nucleotide sequence encoding the Cas protein and/or the nucleotide sequence encoding a suicide gene can be prepared using methods well known in the art. For example, the polynucleotide molecule can be prepared as part of a larger plasmid. Such preparation allows the cloning and isolation of the correct constructs in an efficient manner as is known in the art. The various methods employed in the preparation of the plasmids and transformation of host organisms are known in the art (see, e.g., Molecular Cloning A Laboratory Manual, 2nd Ed., ed. By Sambrook, Fritsch and Maniatis, Cold Spring Harbor Laboratory Press, 1989). In some embodiment, the vector comprises a nucleotide sequence encoding the guide RNA. In some embodiment, the guide RNA comprises a tracr sequence . In some embodiment, the guide RNA and the tracr sequence is expressed in two separate transcripts from one or two vectors. In some embodiments, the nucleotide sequence encoding the guide RNA and that encoding the Cas protein and/or suicide gene can be in the same or different vectors.

The composition as disclosed herein, when being introduced into a target cell, can mediate the integration of the suicide gene at the target sequence, resulting the expression of the suicide gene and causing the death of the target cell. In an embodiment, the suicide gene is integrated to a target sequence specific to the target cell, resulting in the specific killing of the target cell while leaving the cells that do not contain the target sequence alive.

In an embodiment, the suicide gene is operably linked to a regulatory element. In an embodiment, the regulatory element is a promoter. In an embodiment, the regulatory element is a minimal promoter. In an embodiment, said polynucleotide comprising the suicide gene does not comprise a promoter that is operably linked to the suicide gene; in such case, the suicide gene is expressed after incorporating into the target sequence area which is already an active gene transcription area.

The term "operably linked" refers to an arrangement of elements wherein the components so described are configured so as to perform their usual function. In the case of a promoter, a promoter that is operably linked to a coding sequence will direct the expression of the coding sequence. The promoter or other control elements need not be contiguous with the coding sequence, so long as they function to direct the expression thereof. For example, intervening untranslated yet transcribed sequences can be present between the promoter sequence and the coding sequence and the promoter sequence can still be considered "operably linked" to the coding sequence.

A "promoter" refers a DNA sequence that initiates transcription of a particular gene. Non-limiting examples of a promoter commonly used to initiate transcription of a gene in a eukaryotic cell include SV40 promoter, CMV promoter, EF1a promoter, Ubc promoter, human beta actin promoter, CAG promoter and tetracycline response element (TRE) promoter.

In one embodiment, the promoter used in the composition described herein is a minimal promoter that initiates low or no transcription of the suicide gene when the polynucleotide encoding the suicide gene is not integrated in the genome of the target cell. In one example, a minimal or core promoter is a part of promoter located between -35 to +35 region with respect to the transcription start site. It has one or more of 3 conservative sequence i.e. TATA box, initiator region, binding site for RNA polymerase and downstream promoter element. In such example, when being introduced into a target cell, the composition does not kill the target cell unless an integration of the suicide occurs.

In one embodiment, the polynucleotide comprising the suicide gene does not comprise a promoter that is operably linked to the suicide gene. In one example, the target sequence is at the vicinity of an endogenous promoter in the genome of the target cell. When the suicide gene is integrated at the target sequence in the genome, the transcription of the suicide gene is initiated due to the presence of the endogenous promoter. In another example, the target sequence is at a locus of highly active expression (e.g., immunoglobulin locus in a B cell). The suicide gene is highly expressed and kills the target cell only when it is integrated at the high-expression locus. In one example, Chiarle et al. have systematically identified genome-wide translocation of chromosome breaks and rearrangements in B cells and found that translocation preferentially targeted to transcribed chromosomal regions (Cell. 2011, 147(1): 107-19). A target sequence specific to the B cell of translocation is identified on the basis of the DNA sequences surrounding the breakpoint junction of the translocation. When a suicide gene without operably linked to a promoter is integrated into the target sequence, it is expressed and kills the B cell. In contrast, an un-integrated suicide gene does not express even if it is introduced into a cell.

In some embodiments, said target cell is an animal cell. In some embodiments, said target cell is a mammalian cell, e.g., a rodent cell (e.g., a mouse cell) and a human cell. Exemplified target cells encompass epithelial cells, neural cells (e.g., neurons, astrocytes), liver cells, blood cells, adipocytes and kidney cells.

Said target cell is a cancer cell. A cancer cell refers to a cell that grows and divides at unregulated, quickened pace. Examples of cancer include acute lymphoblastic leukemia (ALL), acute myeloid leukemia, adrenocortical carcinoma, anal cancer, astrocytoma, childhood cerebellar or cerebral, basal-cell carcinoma, bile duct cancer, bladder cancer, bone tumor, brain cancer, cerebellar astrocytoma, cerebral astrocytoma/malignant glioma, ependymoma, medulloblastoma, supratentorial primitive neuroectodermal tumors, visual pathway and hypothalamic glioma, breast cancer, Burkitt's lymphoma, ervical cancer, chronic lymphocytic leukemia, chronic myelogenous leukemia, colon cancer, emphysema, endometrial cancer, ependymoma, esophageal cancer, Ewing's sarcoma, retinoblastoma, gastric (stomach) cancer, glioma, head and neck cancer, heart cancer, Hodgkin lymphoma, islet cell carcinoma (endocrine pancreas), Kaposi sarcoma, kidney cancer (renal cell cancer), laryngeal cancer, leukaemia, liver cancer, lung cancer, neuroblastoma, non-Hodgkin lymphoma, ovarian cancer, pancreatic cancer, pharyngeal cancer, prostate cancer, rectal cancer, renal cell carcinoma (kidney cancer), retinoblastoma, Ewing family of tumors, skin cancer, stomach cancer, testicular cancer, throat cancer, thyroid cancer, vaginal cancer

A key issue of cancer therapy is to selectively eliminate cancer cells, which is particularly critical in cases where the cancer cells are intermingled with the healthy cells. The surgical resection unavoidably removes the functional, healthy cells together with the cancer. Radiation therapy affects all the exposed cells. While the radiation therapy relies on the higher sensitivity of rapidly proliferating cells (e.g., cancer cells) to the ionizing radiation, some of the healthy cells, including reproductive, immune, and hormonal systems' cells are also sensitive and thus impaired in the radiation therapy. Likewise, chemotherapeutics penetrate into and affect all the cells. Most chemotherapeutics rely upon the higher uptake rate into rapidly proliferating cells and blocking mitosis, which lead to activating apoptotic cascades. Nevertheless, populations of the healthy cells, including those involved in regeneration and immunity, are also seriously affected.

The composition disclosed therein can be used to selectively eliminate cancer cells. By identifying a target sequence that is specific to a cancer cell, the composition is capable of facilitating the integration of a suicide gene selectively in the cancer cell, causing the cancer cell to kill itself.

In one embodiment, the second nucleotide sequence encoding the Cas protein is operably linked to a cancer-specific promoter. By operably linking the sequence encoding the Cas protein to a cancer-specific promoter, the expression of the Cas protein is restrained to the cancer cells. As a result, the integration of the suicide gene into the genome of a target cell only occurs in the cancer cells. Exemplified examples of cancer-specific promoter encompass epidermal growth factor receptor (EGFR) promoter, transferrin receptor (TfR) promoter, carcinoembryonic antigen (CEA) promoter, telomerase promoter, prostate specific antigen (PSA) promoter, cytokeratin 18 and 19 (CK19) promoter and cyclooxygenase (Cox) promoter.

In another embodiment, the suicide gene is operably linked to a cancer-specific promoter. As such, the integrated suicide gene only express in cancer cell, despite the fact that some off-target integration may occur.

Most human cancers are highly heterogenous. A single cell may harbor multiple mutations in its genome. Therefore, in an aspect, the present application discloses a composition comprising a) a first guide RNA that is capable of hybridizing with a first target sequence in the genome of a target cell; b) a second guide RNA that is capable of hybridizing with a second target sequence in the genome of the target cell; c) a Cas protein; and d) a polynucleotide encoding a suicide gene. Such composition can be used to facilitate integration of a suicide gene into multiple target sequences in the genome of a target cell.

In another aspect, the present application discloses a composition comprising one or more vectors comprising a) a first nucleotide sequence encoding a first guide RNA that is capable of hybridizing with a first target sequence in the genome of a target cell; b) a second nucleotide sequence encoding a second guide RNA that is capable of hybridizing with a second target sequence in the genome of the target cells; c) a third nucleotide sequence encoding a Cas protein; and d) a fourth nucleotide sequence encoding a suicide gene; wherein the first, second, third and fourth nucleotide sequence are in same or different vector.

Cancer heterogeneity not only exists in one cancer cell, but also in a population of cancer cells within a cancer tissue, i.e., different cancer cells can show distinct morphological and phenotypic profiles, including cellular morphology, gene expression, metabolism, motility, proliferation, and metastatic potential. As a result, the heterogeneity of cancer cells introduces significant challenges in designing effective treatment strategies because a cancer therapy that is effective to one cancer cell may not be effective to another cancer cell within the same cancer tissue.

A common feature of cancer heterogeneity is genetic heterogeneity, which can arise form multiple sources. Some cancers are initiated when exogenous factors introduce mutations, such as ultraviolet radiation (e.g., skin cancers) and tobacco (e.g., lung cancer). A more common source is genomic instability, which often arises when key regulatory pathways are disrupted in the cells. Some examples include impaired DNA repair mechanisms, which can lead to increased replication errors, and defects in the mitosis machinery that allow for large-scale gain or loss of entire chromosomes.

Therefore, in an aspect, the present application discloses a composition comprising a) a first guide RNA that is capable of hybridizing with a first target sequence in the genome of a plurality of target cells; b) a second guide RNA that is capable of hybridizing with a second target sequence in the genome of the plurality of target cells; c) a Cas protein; and d) a polynucleotide encoding a suicide gene.

In another aspect, the present application discloses a composition comprising one or more vectors comprising a) a first nucleotide sequence encoding a first guide RNA that is capable of hybridizing with a first target sequence in the genome of a plurality of target cells; b) a second nucleotide sequence encoding a second guide RNA that is capable of hybridizing with a second target sequence in the genome of the plurality of target cells; c) a third nucleotide sequence encoding a Cas protein; and d) a fourth nucleotide sequence encoding a suicide gene; wherein the first, second, third and fourth nucleotide sequence are in same or different vector.

In some embodiments, the composition as described above can be used to kill a population of cells of genetic heterogeneity, i.e., the cells in the population has genomic variations. In one embodiment, the composition as described above can be used to kill cancer cells of genetic heterogeneity. In one example, a biopsy of cancer cells are obtained and the genomes of the cancer cells are sequenced and two or more target nucleotide sequences that are specific in the genomics of the cancer cells as compared to the genome of a healthy cell are identified. Guide RNAs that are capable of hybridizing with the target nucleotide sequences are designed. After being introduced into the cancer cells, the suicide gene is integrated at the target sequences in the genomes of the cancer cells, which results in the death of the cancer cells. As such, the suicide gene is integrated into the genome of a population of cancer cells, despite the genetic heterogeneity in the population, so long as each cancer cell harbors at least one target sequence.

The present application discloses a method comprising introducing into a target cell a composition comprising a) a guide RNA that is capable of hybridizing with a target sequence in the genome of the target cell; b) a Cas protein; and c) a polynucleotide encoding a suicide gene. Said target sequence is specific to the genome of the target cell.

In another aspect, the present application discloses a method comprising contacting a target cell with one or more vectors comprising a) a first nucleotide sequence encoding a guide RNA that is capable of hybridizing with a target sequence in the genome of the target cell; b) a second nucleotide sequence encoding a Cas protein; and c) a third nucleotide sequence encoding a suicide gene; wherein the first, second and third nucleotide sequence are in same or different vector. In an embodiment, said second nucleotide sequence is operably linked to a cancer-specific promoter.

Conventional viral and non-viral based gene transfer methods can be used to introduce the vectors in target cells. Such methods can be used to administer nucleic acids encoding components of the composition to cells in culture, or in a host organism. Non-viral vector delivery systems include DNA plasmids, RNA (e.g. a transcript of a vector described herein), naked nucleic acid, and nucleic acid complexed with a delivery vehicle, such as a liposome, protein complexed with a delivery vehicle, such as a liposome. Viral vector delivery systems include DNA and RNA viruses, which have either episomal or integrated genomes after delivery to the cell. For a review of gene therapy procedures, see Anderson, Science 256:808-813 (1992); Nabel & Felgner, TIBTECH 11:211-217 (1993); Mitani & Caskey, TIBTECH 11:162-166 (1993); Dillon, TIBTECH 11:167-175 (1993); Miller, Nature 357:455-460 (1992); Van Brunt, Biotechnology 6(10): 1149-1154 (1988); Vigne, Restorative Neurology and Neuroscience 8:35-36 (1995); Kremer & Perricaudet, British Medical Bulletin 51(1):31-44 (1995); Haddada et al., in Current Topics in Microbiology and Immunology Doerfler and Bihm (eds) (1995); and Yu et al., Gene Therapy 1:13-26 (1994).

Methods of non-viral delivery of nucleic acids include lipofection, nucleofection, electroporation, microinjection, biolistics, virosomes, liposomes, immunoliposomes, polycation or lipid:nucleic acid conjugates, naked DNA, artificial virions, and agent-enhanced uptake of DNA. Lipofection is described in e.g., U.S. Pat. Nos. 5,049,386, 4,946,787; and 4,897,355) and lipofection reagents are sold commercially (e.g., Transfectam^{™} and Lipofectin^{™}). Cationic and neutral lipids that are suitable for efficient receptor-recognition lipofection of polynucleotides include those of Feigner, WO 91/17424; WO 91/16024. Delivery can be to cells (e.g. in vitro or ex vivo administration) or target tissues (e.g. in vivo administration).

The preparation of lipid:nucleic acid complexes, including targeted liposomes such as immunolipid complexes, is well known to one of skill in the art (see, e.g., Crystal, Science 270:404-410 (1995); Blaese et al., Cancer Gene Ther. 2:291-297 (1995); Behr et al., Bioconjugate Chem. 5:382-389 (1994); Remy et al., Bioconjugate Chem. 5:647-654 (1994); Gao et al., Gene Therapy 2:710-722 (1995); Ahmad et al., Cancer Res. 52:4817-4820 (1992); U.S. Pat. Nos. 4,186,183, 4,217,344, 4,235,871, 4,261,975, 4,485,054, 4,501,728, 4,774,085, 4,837,028, and 4,946,787).

The use of RNA or DNA viral based systems for the delivery of nucleic acids take advantage of highly evolved processes for targeting a virus to specific cells in the body and trafficking the viral payload to the nucleus. Viral vectors can be administered directly to patients (in vivo) or they can be used to treat cells in vitro, and the modified cells may optionally be administered to patients (in vivo). Conventional viral based systems could include retroviral, lentiviral, adenoviral, adeno-associated and herpes simplex viral vectors for gene transfer. Integration in the host genome is possible with the retrovirus, lentivirus, and adeno-associated virus gene transfer methods, often resulting in long term expression of the inserted transgene. Additionally, high transduction efficiencies have been observed in many different cell types and target tissues.

As disclosed herein, the expression of the suicide gene is preferably minimized when it is not integrated into the target sequence. In one embodiment, adenoviral based systems is used because of their low potential of integration into the genome of a target cell in the absence of a CRISPR/Cas system. Adenoviral based vectors are capable of very high transduction efficiency in many cell types and do not require cell division. With such vectors, high titer and levels of expression have been obtained. This vector can be produced in large quantities in a relatively simple system. Adeno-associated virus ("AAV") vectors may also be used to transduce cells with target nucleic acids, e.g., in the in vitro production of nucleic acids and peptides, and for in vivo and ex vivo gene therapy procedures (see, e.g., West et al., Virology 160:38-47 (1987); U.S. Pat. No. 4,797,368; WO 93/24641; Kotin, Human Gene Therapy 5:793-801 (1994); Muzyczka, J. Clin. Invest. 94:1351 (1994). Construction of recombinant AAV vectors are described in a number of publications, including U.S. Pat. No. 5,173,414; Tratschin et al., Mol. Cell. Biol. 5:3251-3260 (1985); Tratschin, et al., Mol. Cell. Biol. 4:2072-2081 (1984); Hermonat & Muzyczka, PNAS 81:6466-6470 (1984); and Samulski et al., J. Virol. 63:03822-3828 (1989).

Packaging cells are typically used to form virus particles that are capable of infecting a host cell. Such cells include 293 cells, which package adenovirus, and ψ2 cells or PA317 cells, which package retrovirus. Viral vectors used in gene therapy are usually generated by producing a cell line that packages a nucleic acid vector into a viral particle. The vectors typically contain the minimal viral sequences required for packaging and subsequent integration into a host, other viral sequences being replaced by an expression cassette for the polynucleotide(s) to be expressed. The missing viral functions are typically supplied in trans by the packaging cell line. For example, AAV vectors used in gene therapy typically only possess ITR sequences from the AAV genome which are required for packaging and integration into the host genome. Viral DNA is packaged in a cell line, which contains a helper plasmid encoding the other AAV genes, namely rep and cap, but lacking ITR sequences. The cell line may also be infected with adenovirus as a helper. The helper virus promotes replication of the AAV vector and expression of AAV genes from the helper plasmid. The helper plasmid is not packaged in significant amounts due to a lack of ITR sequences. Contamination with adenovirus can be reduced by, e.g., heat treatment to which adenovirus is more sensitive than AAV. Additional methods for the delivery of nucleic acids to cells are known to those skilled in the art. See, for example, US20030087817,

In an aspect, the present application discloses a method comprising introducing into a plurality of target cells a composition comprising a) a first guide RNA that is capable of hybridizing with a first target sequence in the genome of the plurality of target cells; b) a second guide RNA that is capable of hybridizing with a second target sequence in the genome of the plurality of target cells; c) a Cas protein; and d) a polynucleotide encoding a suicide gene.

In an aspect, the present application discloses a method comprising contacting a plurality of target cells with one or more vectors comprising a) a first nucleotide sequence encoding a first guide RNA that is capable of hybridizing with a first target sequence in the genome of the plurality of target cells; b) a second nucleotide sequence encoding a second guide RNA that is capable of hybridizing with a second target sequence in the genome of the plurality of target cells; c) a third nucleotide sequence encoding a Cas protein; and d) a fourth nucleotide sequence encoding a suicide gene; wherein the first, second, third and fourth nucleotide sequence are in same or different vector.

In an aspect, the present application discloses a method for treating cancer comprising contacting a cancer cell with one or more vectors comprising a) a first nucleotide sequence encoding a guide RNA that is capable of hybridizing with a target sequence in the genome of the cancer cell; b) a second nucleotide sequence encoding a Cas protein; and c) a third nucleotide sequence encoding a suicide gene; wherein the first, second and third nucleotide sequence are in same or different vector. Said target sequence is specific to the genome of the cancer cell. The method moy further comprise treating cancer cells with prodrugs, e.g., GCV and 5-FC.

In another aspect, the present application disclose a composition comprising a) a gene-targeting or a variant thereof; and b) a polynucleotide encoding a suicide gene; wherein the gene-targeting or its variant is capable of facilitating the integration of the suicide gene into a target sequence in the genome of a target cell. In an embodiment, the gene-targeting nuclease is a zinc-finger nuclease (ZFN), a transcription activator-like effector nuclease (TALEN), a Cas protein, or a meganuclease. The gene-targeting nuclease is capable of creating specific double stranded break at desired locations in the genome (e.g., a target sequence), and harness the cell's endogenous mechanisms to repair the induced break by homologous recombination (HR) and nonhomologous end-joining (NHEJ), resulting in the integration of the suicide gene at the desired locations. In certain embodiments, the variant of gene-targeting nuclease is a gene-targeting recombinase, such as zinc-finger recombinase (ZFR) (Proudfoot et al., (2011) Zinc Finger Recombinases with adaptable DNA specificity, PLoS ONE 6(4):c19537).

In an aspect, the present application discloses a method of treating cancer in a subject suspected to have cancer by introduce to the subject one or more vectors comprising a) a first nucleotide sequence encoding a guide RNA that is capable of hybridizing with a target sequence in the genome of the cancer cell; b) a second nucleotide sequence encoding a Cas protein; and c) a third nucleotide sequence encoding a suicide gene; wherein the first, second and third nucleotide sequence are in same or different vector.

The subject is an animal, e.g., a mammal (e.g., a rodent (e.g., mouse, rat, rabbit), a cat, a dog, a cattle, a primate and a human).

In some embodiments, the method further comprises obtaining a sample of cancer cells from the subject to conduct genome sequencing to identify a target sequence specific to the cancer cells. In some embodiments, the method further comprises administering to the subject a compound that kills the cancer cells with the suicide gene integrated into their genome but does not kill the non-cancer cells. In an example, the compound is a GCV when the suicide gene is HSV-TK. In another example, the compound is 5-FC when the suicide gene is cytosine deaminase.

In an aspect, the present application discloses a guide RNA used in the composition as disclosed herein. In one embodiment, the guide RNA comprises a first nucleotide sequence that complements to a sequence in a first chromosome in a target cell; and a second nucleotide sequence that complements to a sequence in a second chromosome in the target cell. Typically, the first nucleotide sequence is about or more than 5, 6, 7, 8, 9, 10, 11, 12, 13, 14, 15, 16, 17, 18, 19, 20 nucleotide in length, and the second nucleotide sequence is about or more than 5, 6, 7, 8, 9, 10, 11, 12, 13, 14, 15, 16, 17, 18, 19, 20 nucleotide in length. In an embodiment, said guide RNA comprises a nucleotide sequence that complements to a sequence surrounding the breakpoint junction of a chromosome translocation.

In an aspect, the present application discloses a vector comprising (a) a polynucleotide encoding a suicide gene; (b) a first homology arm located at the upstream of the polynucleotide encoding the suicide gene, wherein the first homology arm comprises a sequence in a first chromosome in a target cell; and (c) a second homology arm located at the downstream of the polynucleotide encoding the suicide gene, wherein the second homology arm comprises a sequence in a second chromosome in the target cell. As used herein, a "homology arm" refers to a polynucleotide that suitable to target a gene to a genome through homologous recombination. Typically, two homology arms flank the gene to be integrated into the genome, wherein each homology arm comprises sequences upstream and downstream of the loci of integration. Typically, the first homology arm is about or more than 50, 60, 70, 80, 90, 100, 200, 300, 400, 500, 600, 700, 800, 900, 1000, 1500, 2000 base pair in length, and the second homology arm is at least 50, 60, 70, 80, 90, 100, 200, 300, 400, 500, 600, 700, 800, 900, 1000, 1500, 2000 nucleotide in length.

### EXAMPLE 1

The following is an example of inserting a suicide gene to a target sequence in a cell to induce cell death.

To test the functionality of the strategy disclosed in the present application, HSV-TK is integrated to the AAVS1 (adeno-associated virus integration site 1) locus located in the PPP1R12C gene on chromosome 19 in HEK293T cells.

### Preparation of Cas9 and gRNA expression construct

Plasmid pX330 that contains two expression cassettes, a human codon-optimized *S. pyogenes* Cas9 expression cassette and a U6 promoter driven cassette expressing a single guide RNA, is obtained from Addgene (ID No: 48140). DNA sequences encoding a guide RNA against two Cas9 target, T1 and T2 (see FIG. 2), are cloned into the guide RNA expression cassette of the pX330 plasmid, respectively (see Ran et al. (2013) Genome engineering using the CRISPR-Cas9 system, Nature Protocols 8, 2281-2308).

### Preparation of targeting plasmids containing HSV-TK gene

Targeting plasmids containing HSV-TK gene is prepared using MultiSite Gateway Three-Fragment Vector Construction Kit (Life Technologies) (see Boudabe and Okkenhaug (2013) A protocol for construction of gene targeting vectors and generation of homologous recombination ES cells, Methods Mol Biol. 1064: 337-354). Briefly, 5' homology arm and 3' homology arm corresponding to the sequences surrounding the AAVS1 locus is inserted to the entry vector, respectively. An HSV-TK gene is then inserted to the entry vector containing the 5' homologous arm. The resulting entry vectors are recombined with the destination vector to generate the targeting vector pTarget-HSV-TK-1. As a control, a targeting vector with GFP (green fluorescence protein) gene flanked by the 5' and 3' homologous arm is generated (pTarget-GFP). The control targeting vector also contains a protospacer 2 sequence at the 3' downstream of the GFP gene and following an IRES (internal ribosome entry site) sequence. The targeting plasmids are linearized by cutting once at a restriction site in the vector backbone near one of the homology arms.

### Co-transfection of CRISPR plasmids and donor DNA into HEK 293T cells

HEK 293T cells (Life Technologies) are maintained according to the manufacturer's recommendation and cultured in D10 medium (DMEM medium supplemented with GlutaMAX and 10% (vol/vol) FBS) at 37°C and 5% CO₂. Mix the CRISPR plasmids (500ng) and linearized targeting plasmids (500 ng) and prepare the transfection solution using the Amaxa SF cell line 4D-Nucleofector X kit S (Lonza). Add the transfection solution to the cells and electroporate the cells by using the Amaxa, CM-130 (Lonza).

### Insertion of the suicide gene to the AAVS1 locus

Three days after the co-transfection, G418 (400 ug/ml) is added to the cell culture to select the cells containing the targeting vector. After 7 days of selection, cells are cloned and expanded. The sequence at the AAVS1 locus of each clone is determined by PCR or sequencing to confirm the insertion of the HSV-TK gene. The results show that both T1 and T2 gRNA can induce homologous recombination and insertion of HSV-TK gene to the AAVS1 locus. For the control targeting vector, cells with GFP gene inserted at AAVS1 are cloned using the same protocol.

### Selectively eliminating cells inserted with suicide gene

For each of the clones containing HSV-TK insertion, ganciclovir (0.5ug/ml) is added. Three days later, no living cell is found in the HSV-TK containing clones. As a control, ganciclovir does not induce cell death in cells with insertion of GFP gene. To demonstrate selectively eliminating cells inserted with suicide gene, cells containing HSV-TK insertion are mixed with cells containing GFP gene. After treatment of ganciclovir, only GFP-expressing cells survive in the cell culture.

### EXAMPLE 2

The following is an example of selectively eliminating cells of interest. In this example, HSV-TK gene is inserted to cells containing protospacer sequence and induces the cell death.

The cell inserted with the GFP gene as disclosed in Example 1 also contains at the downstream of the GFP gene a protospacer 2 sequence (SEQ ID NO.: 5), which is used in this Example for inserting a suicide gene. Guide RNA is designed according to the protospacer 2 sequence and inserted to the pX330 vector to generate the CRISPR plasmids pX330-p. The targeting vector is constructed as disclosed in Example 1 and contains a HSV-tk gene flanked with a 5' homologous arm consists of GFP gene and the same 3' homologous arm as being used in Example 1. As a result, if the target vector is inserted through homologous recombination, the GFP expression would not be disrupted.

To show selective elimination of cells containing the protospacer sequence, HEK 293T cells with GFP-protospacer sequence inserted (see Example 1) are mixed with unmodified HEK 293T cells. The mixed cell population are split into two dishes. One dish is co-transfected with CRISPR plasmids pX330-p and the targeting plasmids pTarget-HSV-TK-2. The other dish as control is co-transfected with pX330 and the targeting plasmids pTarget-HSV-TK-2. Three days later, ganciclovir is added to the dishes. The number of GFP expressing cells in each dish is counted using FACS the next day. It is observed that in the dish transfected with pX330-p, the percentage of GFP expressing cells is significantly decreased, indicating that cells containing the protospacer sequence are selectively eliminated.

### EXAMPLE 3

The following is an example of eliminating cancer cells in a subject of Philadelphia positive acute lymphoblastic leukemia (ALL).

The most common chromosomal aberration observed in adult ALL is the translocation t(9;22)(q34;q11). The breakpoint junction fragments from the DNA of one patient was characterized by cloning and restriction mapping (Nucleic Acids Res. Jan 1989, 17(1): 1-10). The junction was localized in the intron of ABL between exon Ia and the common exon (a2). The breakpoint on the chromosome 22 was located in the first intron. The sequence surrounding the junction was determined (FIG. 3) and analyzed using a CRISPR gRNA design tool (DNA2.0). A potential guide sequence was identified (FIG. 3).

The blood sample of the subject is obtained. The sequence surrounding the breakpoint junction is characterized by PCR and sequencing. A guide sequence is designed based on the sequence surrounding the breakpoint junction. The guide sequence is cloned into an adeno-associated viral (AAV)/CRISPR vector (Senis E et al., Biotechnol J. 2014, 9: 1402-12). The cloned AAV/CRISPR vectors are packaged together with a AAV/HSV-TK vector (Kanazawa T et al., Gene Ther 2003, 10: 51-58) into recombinant AAV. The AAV/HSV-TK vector contains two homology arms franking the HSV-TK gene, whereas each homology arm comprises sequences immediately upstream and downstream of the breakpoint junction. The HSV-TK in the AAV vector is operably linked to the minimal promoter to ensure that its expression in the absence of integration into the loci of breakpoint junction is minimalized. The recombinant AAV is then delivered into the subject. The blood sample of the subject is obtained to confirm the integration and expression of HSV-TK gene at the loci of the breakpoint junction in Philadelphia positive cancer cells. Ganciclovir (GCV) is then administered to the subject. Cell death of Philadelphia positive cancer cells is observed, while healthy cells are intact.

## Claims

1. A composition for use in a method of treating cancer by selectively killing a cancer cell, comprising
a) a guide RNA that is capable of hybridizing with a target sequence in the genome of the cancer cell, wherein the target sequence is specific to the cancer cell;
b) a Cas protein; and
c) a polynucleotide encoding a suicide gene;
wherein the Cas protein is capable of facilitating the integration of the suicide gene into the target sequence in the genome of the cancer cell.

2. The composition for use of claim 1,
wherein the target sequence comprises a breakpoint junction of a chromosome translocation in the cancer cell; or
wherein the Cas protein is a Cas9 protein; or
wherein the suicide gene is selected from the group consisting of viral thymidine kinase, cytosine deaminases, intracellular antibody against antioxidative enzymes, bacterial nitroreductase, caspase and DNase; or
wherein the suicide gene is operably linked to a regulatory element, preferably wherein the regulatory element is a cancer-specific promoter.

3. A composition for use in a method of treating cancer by selectively killing a cancer cell, comprising one or more vectors comprising
a) a first nucleotide sequence encoding a guide RNA that is capable of hybridizing with a target sequence in the genome of the cancer cell, wherein the target sequence is specific to the cancer cell;
b) a second nucleotide sequence encoding a Cas protein; and
c) a third nucleotide sequence encoding a suicide gene;
wherein the first, second and third nucleotide sequence are in same or different vector; and
wherein the Cas protein is capable of facilitating the integration of the suicide gene into the target sequence in the genome of the cancer cell;
wherein the cancer cell is not a prostate cancer cell.

4. The composition for use of claim 3, wherein the second nucleotide sequence encoding the Cas protein is operably linked to a cancer-specific promoter.

5. The composition for use according to any of claims 1-4, wherein the method comprises contacting the cancer cell with the composition.

6. The composition for use according to claim 4, wherein the method further comprises administering a prodrug of the suicide gene.

7. A cell comprising the composition of any of claims 1-6.

8. A composition for use in a method of treating cancer by selectively killing a cancer cell, comprising
a) a gene-targeting nuclease; and
b) a polynucleotide encoding a suicide gene;
wherein the gene-targeting nuclease is capable of facilitating the integration of the suicide gene into a target sequence in the genome of a cancer cell, wherein the target sequence is specific to the cancer cell;
preferably, wherein the gene-targeting nuclease is a zinc-finger nuclease (ZFN), a transcription activator-like effector nuclease (TALEN), a Cas protein, or a meganuclease.

9. One or more vectors comprising,
a) a first nucleotide sequence encoding a guide RNA that is capable of hybridizing with a target sequence specific to the genome of a cancer cell;
b) a second nucleotide sequence encoding a Cas protein; and
c) a third nucleotide sequence encoding a suicide gene;
for use in treating cancer, wherein the first, second and third nucleotide sequence are in same or different vector, and wherein the Cas protein is capable of facilitating the integration of the suicide gene into the target sequence in the genome of the target cell;
wherein the cancer cell is not a prostate cancer cell.

10. The one or more vectors for use of claim 9,
further comprising administering the subject a prodrug of the suicide gene.

11. An in vitro method for selectively eliminating a first cell in a cell population comprising at least the first cell and a second cell, the method comprising:
a) identifying a locus in a genome of the first cell, said locus comprising a polynucleotide sequence, wherein the second cell does not comprise the polynucleotide sequence in its genome; and
b) integrating a suicide gene at the locus to induce the cell death of the first cell;
preferably wherein the first cell is a cancer cell.

## Patentansprüche

1. Zusammensetzung zur Verwendung in einem Verfahren zum selektiven Abtöten einer Krebszelle, welche umfasst,
a) eine Guide-RNA, die geeignet ist, mit einer Zielsequenz in dem Genom der Krebszelle zu hybridisieren, worin die Zielsequenz für die Krebszelle spezifisch ist;
b) ein Cas-Protein; und
c) ein Polynukleotid, das ein Suicide-Gen kodiert;
worin das Cas-Protein geeignet ist, die Integration des Suicide-Gens in die Zielsequenz in dem Genom der Krebszelle zu fördern.

2. Zusammensetzung zur Verwendung nach Anspruch 1,
worin die Zielsequenz eine Bruchstellenverbindung einer Chromosomen-Translokation in der Krebszelle umfasst, oder
worin das Cas-Protein ein Cas9-Protein ist; oder
worin das Suicide-Gen ausgewählt ist, aus der Gruppe bestehend aus viraler Thymidin-Kinase, Cytosin-Deaminasen, intrazellulären Antikörpern gegen antioxidative Enzyme, bakterieller Nitroreduktase, Caspase und DNase; oder
worin das Suicide-Gen mit einem regulatorischen Element operativ verbunden ist, vorzugsweise worin das regulatorische Element ein Krebs-spezifischer Promoter ist.

3. Zusammensetzung zur Verwendung in einem Verfahren zum selektiven Abtöten einer Krebszelle, die einen oder mehrere Vektoren umfasst, welche umfassen,
a) eine erste Nukleotidsequenz, die eine Guide-RNA codiert, die geeignet ist, mit einer Zielsequenz in dem Genom der Krebszelle zu hybridisieren, worin die Zielsequenz für die Krebszelle spezifisch ist;
b) eine zweite Nukleotidsequenz, die ein Cas-Protein codiert; und
c) eine dritte Nukleotidsequenz, die ein Suicide-Gen codiert;
worin die erste, zweite und dritte Nukleotidsequenz in dem gleichen oder verschiedenen Vektoren sind; und
worin das Cas-Protein geeignet ist, die Integration des Suicide-Gens in die Zielsequenz in dem Genom der Krebszelle zu fördern;
worin die Krebszelle keine Prostatakrebszelle ist.

4. Zusammensetzung zur Verwendung nach Anspruch 3, worin die zweite das Cas-Protein codierende Nukleotidsequenz mit einem Krebs-spezifischen Promoter operativ verbunden ist.

5. Zusammensetzung zur Verwendung nach einem der Ansprüche 1-4, worin das Verfahren ein In-Kontakt-Kommen der Krebszelle mit der Zusammensetzung umfasst.

6. Zusammensetzung zur Verwendung nach Anspruch 4, wobei das Verfahren weiter Verabreichen einer Prodrug des Suicide-Gens umfasst.

7. Zelle, welche die Zusammensetzung nach einem der Ansprüche 1-6 umfasst.

8. Zusammensetzung zur Verwendung in einem Verfahren zum selektiven Abtöten einer Krebszelle, welche umfasst,
a) eine Gen-*targeting*-Nuklease; und
b) ein Polynukleotid, das ein Suicide-Gen codiert;
worin die Gen-*targeting*-Nuklease geeignet ist, die Integration des Suicide-Gens in eine Zielsequenz in dem Genom einer Krebszelle zu fördern, worin die Zielsequenz für die Krebszelle spezifisch ist;
vorzugsweise, worin die Gen-*targeting*-Nuklease eine Zink-Finger-Nuklease (ZFN), ein Transkriptions-Aktivator-ähnliche Effektor-Nuklease (TALEN), ein Cas-Protein, oder eine Meganuklease ist.

9. Ein oder mehrere Vektoren, welche umfassen,
a) eine erste Nukleotidsequenz, die eine Guide-RNA codiert, die geeignet ist, mit einer für das Genom einer Krebszelle spezifischen Zielsequenz zu hybridisieren;
b) eine zweite Nukleotidsequenz, die ein Cas-Protein codiert; und
c) eine dritte Nukleotidsequenz, die ein Suicide-Gen codiert;
zur Verwendung bei der Behandlung von Krebs, worin die erste, zweite und dritte Nukleotidsequenz in dem gleichen oder einem verschiedenen Vektor sind, und worin das Cas-Protein geeignet ist, die Integration des Suicide-Gens in die Zielsequenz in dem Genom der Krebszelle zu fördern;
worin die Krebszelle keine Prostatakrebszelle ist.

10. Ein oder mehrere Vektoren zur Verwendung nach Anspruch 9,
welche weiter ein Verabreichen einer Prodrug des Suicide-Gens an die Person umfassen.

11. In-vitro-Verfahren zum selektiven Entfernen einer ersten Zelle in einer Zellpopulation, die mindestens die erste Zelle und eine zweite Zelle umfasst, wobei das Verfahren umfasst:
a) Identifizieren eines Locus in einem Genom der ersten Zelle, worin der Locus eine Polynukleotidsequenz umfasst, worin die zweite Zelle die Polynukleotidsequenz nicht in ihrem Genom umfasst, und
b) Integrieren eines Suicide-Gens an dem Locus, um den Zelltod der ersten Zelle zu induzieren;
vorzugsweise worin die erste Zelle eine Krebszelle ist.

## Revendications

1. Composition pour une utilisation dans un procédé de destruction sélective d'une cellule cancéreuse, comprenant
a) un ARN guide qui est capable de s'hybrider avec une séquence cible dans le génome de la cellule cancéreuse, la séquence cible étant spécifique à la cellule cancéreuse ;
b) une protéine Cas ; et
c) un polynucléotide codant pour un gène suicide ;
la protéine Cas étant capable de faciliter l'intégration du gène suicide dans la séquence cible dans le génome de la cellule cancéreuse.

2. Composition pour une utilisation selon la revendication 1,
la séquence cible comprenant une jonction de rupture d'une translocation chromosomique dans la cellule cancéreuse ; ou
la protéine Cas étant une protéine Cas9 ; ou
le gène suicide étant choisi dans le groupe constitué par la thymidine kinase virale, les cytosine désaminases, un anticorps intracellulaire contre les enzymes antioxydantes, une nitroréductase bactérienne, une caspase et une ADNase ; ou
le gène suicide étant lié de manière fonctionnelle à un élément régulateur, préférablement, l'élément régulateur étant un promoteur spécifique au cancer.

3. Composition pour une utilisation dans un procédé de destruction sélective d'une cellule cancéreuse, comprenant un ou plusieurs vecteurs comprenant
a) une première séquence nucléotidique codant pour un ARN guide qui est capable de s'hybrider avec une séquence cible dans le génome de la cellule cancéreuse, la séquence cible étant spécifique à la cellule cancéreuse ;
b) une deuxième séquence nucléotidique codant pour une protéine Cas ; et
c) une troisième séquence nucléotidique codant pour un gène suicide ;
la première, la deuxième et la troisième séquence nucléotidique étant dans le même vecteur ou dans un vecteur différent ; et
la protéine Cas étant capable de faciliter l'intégration du gène suicide dans la séquence cible dans le génome de la cellule cancéreuse ;
la cellule cancéreuse n'est pas une cellule cancéreuse de la prostate.

4. Composition pour une utilisation selon la revendication 3, la deuxième séquence nucléotidique codant pour la protéine Cas étant fonctionnellement liée à un promoteur spécifique au cancer.

5. Composition pour une utilisation selon l'une quelconque des revendications 1 à 4, le procédé comprenant la mise en contact de la cellule cancéreuse avec la composition.

6. Composition pour une utilisation selon la revendication 4, le procédé comprenant en outre l'administration d'un promédicament du gène suicide.

7. Cellule comprenant la composition selon l'une quelconque des revendications 1 à 6.

8. Composition pour une utilisation dans un procédé de destruction sélective d'une cellule cancéreuse, comprenant
a) une nucléase ciblant un gène ; et
b) un polynucléotide codant pour un gène suicide ;
la nucléase ciblant un gène étant capable de faciliter l'intégration du gène suicide dans une séquence cible dans le génome d'une cellule cancéreuse, la séquence cible étant spécifique à la cellule cancéreuse ;
préférablement, la nucléase ciblant un gène étant une nucléase à doigt de zinc (ZFN), une nucléase effectrice de type activateur de transcription (TALEN), une protéine Cas ou une méganucléase.

9. Un ou plusieurs vecteurs comprenant
a) une première séquence nucléotidique codant pour un ARN guide qui est capable de s'hybrider avec une séquence cible spécifique au génome d'une cellule cancéreuse ;
b) une deuxième séquence nucléotidique codant pour une protéine Cas ; et
c) une troisième séquence nucléotidique codant pour un gène suicide ;
pour une utilisation dans le traitement du cancer, la première, la deuxième et la troisième séquence nucléotidique étant dans le même vecteur ou dans un vecteur différent et la protéine Cas étant capable de faciliter l'intégration du gène suicide dans la séquence cible dans le génome de la cellule cancéreuse ;
la cellule cancéreuse n'est pas une cellule cancéreuse de la prostate.

10. Un ou plusieurs vecteurs pour une utilisation selon la revendication 9, comprenant en outre l'administration, au sujet, d'un promédicament du gène suicide.

11. Procédé in vitro pour éliminer sélectivement une première cellule dans une population de cellules comprenant au moins la première cellule et une deuxième cellule, le procédé comprenant :
a) l'identification d'un locus dans un génome de la première cellule, ledit locus comprenant une séquence polynucléotidique, la deuxième cellule ne comprenant pas la séquence polynucléotidique dans son génome ; et
b) l'intégration d'un gène suicide au niveau du locus pour induire la mort cellulaire de la première cellule ; préférablement, la première cellule étant une cellule cancéreuse.


### SEQUENCE LISTING

<110> ZHU, James
<120> METHODS AND COMPOSITIONS FOR SELECTIVELY ELIMINATING CELLS OF INTEREST
<130> 063148-8002WO01
<150> 62/091,431
   <151> 2014-12-12
<160> 5
<170> PatentIn version 3.5
<210> 1
   <211> 1368
   <212> PRT
   <213> Streptococcus pyogenes
<400> 1
<210> 2
   <211> 4104
   <212> DNA
   <213> Artificial Sequence
<220>
   <223> Cas 9 gene
<400> 2
   atggacaaga agtatagcat cgggctggac attggaacga actcggttgg ttgggctgtg      60
   attacggacg aatacaaggt gccatccaag aagtttaagg tcctgggaaa caccgaccgt     120
   cactcaatca agaagaatct cattggagcc ctgctcttcg atagtgggga gaccgccgaa     180
   gctactcgac tgaagcgaac ggctcgccgg cgttatacac gacgcaagaa tcgcatctgc     240
   tacctccagg agattttcag caacgaaatg gctaaggttg atgactcatt ctttcatcga     300
   ctcgaagaaa gtttcttggt cgaggaggat aagaagcacg agcgccatcc gatctttggt     360
   aacattgtgg atgaggttgc ctatcacgaa aagtacccaa ctatctatca tcttcgtaag     420
   aagctggtcg atagcacgga caaggctgat ttgcgactta tctacctggc actcgcgcac     480
   atgattaagt tccgcggcca ttttcttatc gagggtgacc tgaaccccga taattctgac     540
   gttgataagc tcttcatcca gttggtccaa acctacaatc agctgtttga ggaaaaccct     600
   attaatgcat ctggcgtgga cgccaaggct atcctttcgg cgcgcctgtc taagtcgcgg     660
   cgtttggaga accttatcgc acaactcccc ggcgaaaaga agaacggcct cttcggtaat     720
   ttgattgcgt tgtcacttgg tctgactcct aacttcaaga gtaattttga cctggcagag     780
   gatgcgaagc tccagttgtc taaggatacg tatgatgacg atctcgacaa cttgcttgcc     840
   caaatcggtg accagtacgc tgatcttttc ctggccgcta agaatctctc agatgcaatc     900
   ctgctcagtg acattttgcg ggtcaacacc gagattacta aggcccccct gtcagctagt      960
   atgatcaagc ggtatgatga gcaccatcag gacctcacct tgcttaaggc cctcgtgcgt    1020
   cagcaattgc ctgagaagta caaggaaatc ttctttgacc aatccaagaa cggatacgca    1080
   gggtatattg atggcggtgc gagccaggag gaattctaca agtttatcaa gccgattttg    1140
   gagaagatgg acggcactga ggaactgctc gtcaagctga atcgcgaaga tttgcttcgt    1200
   aagcaacgaa cgttcgacaa cggctccatc ccgcaccaga ttcatctggg cgagctccac    1260
   gccatccttc gacgccagga agatttctac ccatttctga aggacaaccg tgagaagatc    1320
   gaaaagattc ttacattccg aatcccctac tatgtgggac ctttggcccg tgggaattcc    1380
   cgatttgctt ggatgacccg aaagagcgag gaaaccatca ctccgtggaa cttcgaggaa    1440
   gtcgtggaca agggtgcatc cgcgcagagc ttcattgagc ggatgaccaa ttttgataag    1500
   aaccttccga atgaaaaggt cctgccaaag cattcgctgc tctacgagta tttcaccgtg    1560
   tataacgaac tgactaaggt caagtacgtg acggagggaa tgcggaagcc agccttcctc    1620
   tcaggggaac aaaagaaggc tatcgtcgat ttgcttttta agaccaatcg taaagtgact    1680
   gttaagcagc tgaaggagga ttatttcaag aagattgaat gtttcgactc cgtcgagatc    1740
   agcggcgtgg aagatcgctt taacgcttcc ctcggtacct accacgacct gctcaagatc    1800
   attaaggaca aggatttcct cgataacgag gaaaatgagg acatcttgga agatattgtc    1860
   ctcacgttga cactttttga ggaccgcgaa atgatcgagg aacggctcaa gacatatgcc 1920
   catttgttcg acgataaggt gatgaagcag ctgaagcggc gtcgatacac cggatggggt 1980
   cgccttagcc ggaagctgat caacggcatt cgagataagc aatctggtaa gactatcttg 2040
   gatttcctta agtcggacgg cttcgccaac cgcaatttta tgcagcttat tcacgacgat 2100
   tccctgacgt tcaaggagga catccagaag gcacaagtct caggacaagg ggattccctg 2160
   cacgagcata tcgccaacct ggctggatcc ccggcgatca agaaggggat tcttcagacc 2220
   gtcaaggttg tcgacgagct ggtcaaggtg atgggccgtc ataagccaga aaacatcgtg 2280
   attgagatgg cccgagaaaa tcagaccact caaaagggtc agaagaacag ccgcgagcgg 2340
   atgaagcgga tcgaggaagg cattaaggaa cttggttctc agatcctgaa ggagcaccct 2400
   gttgaaaaca cacagctcca aaatgagaag ctgtatctct actatttgca aaatggacgc 2460
   gacatgtacg tcgatcagga gctcgacatt aaccggttgt cggactacga tgttgaccat 2520
   atcgtcccgc aatccttcct taaggacgat agcattgata acaaggtgct gactcgctca 2580
   gataagaacc ggggcaagtc cgacaatgtt ccaagcgagg aagtggttaa gaagatgaag 2640
   aactactggc gccaattgct taatgccaag ctcatcacac agcgcaagtt tgacaacttg 2700
   accaaggccg agcggggagg gctgagtgaa ctcgataagg ctggcttcat caagcgtcaa 2760
   ctcgtggaga cgcgacagat cacaaagcac gttgctcaga ttctggactc ccggatgaac 2820
   acaaagtacg acgagaatga taagctcatc cgtgaagtta aggtcattac cctcaagtct 2880
   aagttggtgt cggatttccg caaggacttc caattttata aggttcggga gatcaacaat 2940
   tatcaccatg cacatgatgc gtacctcaac gcagtcgtgg gaactgcgct catcaagaag 3000
   tatcccaagt tggagtccga attcgtctac ggggattata aggtttacga cgtccgcaag 3060
   atgatcgcca agagtgagca ggaaattggc aaggccacgg ctaagtattt cttttactcc 3120
   aacatcatga atttctttaa gacggagatc acactcgcca atggagaaat ccgtaagcga 3180
   cctttgattg agaccaacgg cgagactggt gaaatcgttt gggataaggg gcgcgacttc 3240
   gctaccgtgc ggaaggttct gagcatgccg caagtcaata tcgtcaagaa aaccgaggtg 3300
   cagacaggcg gtttctctaa ggaatcgatt cttccaaagc gtaactctga caagctgatc 3360
   gctcgaaaga aggattggga ccccaagaag tatggagggt tcgattctcc tacagtggca 3420
   tactcggttc tcgttgtcgc gaaggttgag aagggaaagt ctaagaagct gaagtcggtc 3480
   aaggaactgc tcgggatcac cattatggag cgctccagct tcgaaaagaa tcccatcgac 3540
   tttctcgagg ccaagggcta taaggaagtc aagaaggatc ttatcattaa gctgcctaag 3600
   tactctttgt tcgagcttga aaacggtcga aagcgaatgc tcgcatcggc aggagagttg 3660
   cagaagggga atgaattggc acttccctca aagtacgtga acttcctgta tctcgcgtcc 3720
   cactacgaga agctgaaggg tagccctgag gacaacgaac agaagcaact ttttgttgag     3780
   caacacaagc attatctgga tgagatcatt gaacagattt cagagttcag taagcgcgtc     3840
   atcctcgccg atgctaatct cgacaaggtg ttgtcggcct acaacaagca ccgtgacaag     3900
   ccgatccgag agcaggctga aaatatcatt catctgttca ccctcactaa cttgggagca     3960
   ccagcagcgt tcaagtattt tgatacgaca atcgaccgta agcgatacac gtccacaaag     4020
   gaggtgcttg atgcgaccct gattcatcaa tccatcactg ggctctatga aacccgtatc     4080
   gaccttagtc aactgggggg cgac                                            4104
<210> 3
   <211> 376
   <212> PRT
   <213> Artificial Sequence
<220>
   <223> Thymidine kinase derived from Herpes simplex virus
<400> 3
   Met Ala Ser Tyr Pro Gly His Gln His Ala Ser Ala Phe Asp Gln Ala
   1               5                   10                  15
   Ala Arg Ser Arg Gly His Ser Asn Arg Arg Thr Ala Leu Arg Pro Arg
               20                  25                  30
   Arg Gln Gln Glu Ala Thr Glu Val Arg Pro Glu Gln Lys Met Pro Thr
           35                  40                  45
   Leu Leu Arg Val Tyr Ile Asp Gly Pro His Gly Met Gly Lys Thr Thr
       50                  55                  60
   Thr Thr Gln Leu Leu Val Ala Leu Gly Ser Arg Asp Asp Ile Val Tyr
   65                  70                  75                  80
   Val Pro Glu Pro Met Thr Tyr Trp Arg Val Leu Gly Ala Ser Glu Thr
                   85                  90                  95
   Ile Ala Asn Ile Tyr Thr Thr Gln His Arg Leu Asp Gln Gly Glu Ile
               100                 105                 110
   Ser Ala Gly Asp Ala Ala Val Val Met Thr Ser Ala Gln Ile Thr Met
           115                 120                 125
   Gly Met Pro Tyr Ala Val Thr Asp Ala Val Leu Ala Pro His Ile Gly
       130                 135                 140
   Gly Glu Ala Gly Ser Ser His Ala Pro Pro Pro Ala Leu Thr Leu Ile
   145                 150                 155                 160
   Phe Asp Arg His Pro Ile Ala Ala Leu Leu Cys Tyr Pro Ala Ala Arg
                   165                 170                 175
   Tyr Leu Met Gly Ser Met Thr Pro Gln Ala Val Leu Ala Phe Val Ala
               180                 185                 190
   Leu Ile Pro Pro Thr Leu Pro Gly Thr Asn Ile Val Leu Gly Ala Leu
           195                 200                 205
   Pro Glu Asp Arg His Ile Asp Arg Leu Ala Lys Arg Gln Arg Pro Gly
       210                 215                 220
   Glu Arg Leu Asp Leu Ala Met Leu Ala Ala Ile Arg Arg Val Tyr Gly
   225                 230                 235                 240
   Leu Leu Ala Asn Thr Val Arg Tyr Leu Gln Cys Gly Gly Ser Trp Arg
                   245                 250                 255
   Glu Asp Trp Gly Gln Leu Ser Gly Thr Ala Val Pro Pro Gln Gly Ala
               260                 265                 270
   Glu Pro Gln Ser Asn Ala Gly Pro Arg Pro His Ile Gly Asp Thr Leu
           275                 280                 285
   Phe Thr Leu Phe Arg Ala Pro Glu Leu Leu Ala Pro Asn Gly Asp Leu
       290                 295                 300
   Tyr Asn Val Phe Ala Trp Ala Leu Asp Val Leu Ala Lys Arg Leu Arg
   305                 310                 315                 320
   Ser Met His Val Phe Ile Leu Asp Tyr Asp Gln Ser Pro Ala Gly Cys
                   325                 330                 335
   Arg Asp Ala Leu Leu Gln Leu Thr Ser Gly Met Val Gln Thr His Val
               340                 345                 350
   Thr Thr Pro Gly Ser Ile Pro Thr Ile Cys Asp Leu Ala Arg Thr Phe
           355                 360                 365
   Ala Arg Glu Met Gly Glu Ala Asn
       370                 375
<210> 4
   <211> 1133
   <212> DNA
   <213> Artificial Sequence
<220>
   <223> HSV-tk nucleotide sequence
<400> 4
   gtatggcttc gtaccccggc catcaacacg cgtctgcgtt cgaccaggct gcgcgttctc      60
   gcggccatag caaccgacgt acggcgttgc gccctcgccg gcagcaagaa gccacggaag     120
   tccgcccgga gcagaaaatg cccacgctac tgcgggttta tatagacggt ccccacggga     180
   tggggaaaac caccaccacg caactgctgg tggccctggg ttcgcgcgac gatatcgtct     240
   acgtacccga gccgatgact tactggcggg tgctgggggc ttccgagaca atcgcgaaca     300
   tctacaccac acaacaccgc ctcgaccagg gtgagatatc ggccggggac gcggcggtgg     360
   taatgacaag cgcccagata acaatgggca tgccttatgc cgtgaccgac gccgttctgg     420
   ctcctcatat cgggggggag gctgggagct cacatgcccc gcccccggcc ctcaccctca     480
   tcttcgaccg ccatcccatc gccgccctcc tgtgctaccc ggccgcgcgg taccttatgg     540
   gcagcatgac cccccaggcc gtgctggcgt tcgtggccct catcccgccg accttgcccg     600
   gcaccaacat cgtgcttggg gcccttccgg aggacagaca catcgaccgc ctggccaaac     660
   gccagcgccc cggcgagcgg ctggacctgg ctatgctggc tgcgattcgc cgcgtttacg     720
   ggctacttgc caatacggtg cggtatctgc agtgcggcgg gtcgtggcgg gaggactggg     780
   gacagctttc ggggacggcc gtgccgcccc agggtgccga gccccagagc aacgcgggcc     840
   cacgacccca tatcggggac acgttattta ccctgtttcg ggcccccgag ttgctggccc     900
   ccaacggcga cctgtataac gtgtttgcct gggccttgga cgtcttggcc aaacgcctcc     960
   gttccatgca cgtctttatc ctggattacg accaatcgcc cgccggctgc cgggacgccc    1020
   tgctgcaact tacctccggg atggtccaga cccacgtcac cacccccggc tccataccga    1080
   cgatatgcga cctggcgcgc acgtttgccc gggagatggg ggaggctaac tga           1133
<210> 5
   <211> 23
   <212> DNA
   <213> Artificial Sequence
<220>
   <223> protospacer2
<400> 5
   ataactcaat ttgtaaaaaa ggg 23